# EUROPEAN PATENT APPLICATION

(11) **EP 1 731 108 A1**
(43) Date of publication of application: **13.12.2006**
(21) Application number: 05020096.3
(22) Date of filing: 15.09.2005
(51) Int. Cl.: A61B 17/82, A61B 17/04

(54) **Metallic clamp for surgical applications**

(30) Priority: 06.06.2005 BR 0501134 U
(71) Applicant: De Oliveira, Joao Bosco, 05657-230 Sao Paulo (BR)
(72) Inventor: De Oliveira, Joao Bosco, 05657-230 Sao Paulo (BR)
(74) Representative: Fernandez Lerroux, Aurelio

(57) **Abstract**

A metallic clamp (1), also defined as metallic tape, of ordinary use during invasive surgical interventions, which receives a guide-wire element (1D), more specifically a guide-wire element fixed to one edge of the metallic clamp, aiding in a decisive way the procedure of adequate placement close to osseous structure to be closed at the end of the surgical intervention. The metallic clamp provides for ergonomics application, producing reliability gains for a surgical procedure as a whole, bringing a technical and operational alternative to the use of steel tape elements.

## Description

### FIELD OF THE INVENTION

The present invention is related to clamps used on medical procedures and for surgical applications.

### BACKGROUND OF THE INVENTION

The field of medicine, particularly surgical medicine, has experienced a great evolution since last century, creating original techniques of surgical interventions, as the ones applied to cardiac surgeries.

An important characteristic of this kind of surgery is the highly invasive procedure, needing the incision of the thorax bone called "sternum", provoking a traumatism of sore recovery to the patient.

According to the point of view of a surgical team, the procedure of tissue suture and incised osseous structure recovery, reparation and reconstruction presents a great level of physical and mental stress.

Particularly, the invasive surgical procedure requires the use of special instruments, suitable and fitted to cut and separate tissues during the procedures and invasion. To this specific aim and objective, instruments such as scalpels, scissors, forceps, separators, tweezers and saws are very helpful.

When performing a cutting and sawing procedure of tissues, muscles and osseous structures, the reverse procedure becomes obligatory. These procedures are known as structure reparation of traumatized structures during the surgical intervention and the medical team uses several elements as needles, needle chuck, wires and/or suture tapes that may be or not reabsorbed by the human body.

According to the scope of non-reabsorbable elements by the human body, the present invention enhances the conditions to execute the procedure of osseous structure restoring, using again the example of the restoring of "sternum" bone, which forms the thoracic cavity of human skeleton.

In the mentioned procedure the medical team uses surgical steel wires or tapes that, besides having natural asepsis characteristics and histocompatibility, must present a great efficiency of the main function: to provide the union of the separated parts of the osseous structure.

Inside this context, the applicant takes as a parameter of performance improvement study the surgical steel tape elements in detriment of the mentioned steel wires of same nature, since the last ones are only used to healthy osseous structures, namely, structures with no wearing or structural weakness, characteristic of pathologies as osteoporosis.

On the other hand, the use of surgical steel tapes during osseous structure restoring, which was cut during a surgical intervention, is hardly done in an isolated way with the fix of a sole tape unity, being habitual the use of at least a set of tapes.

Nevertheless, a most deep analysis of the value related to the efficiency of fixing procedure of such steel tape leads to a conclusion that the surgeon hardly places the tape involved with the separated parts of the "sternum" bone, for example, being conclusive that the steel tape product presents ergonomics deficiency of application.

According to the difficulty degree expressed on the study, it is possible to affirm the ergonomics deficiency of clamps and applications known in the art takes to a non-favorable condition to surgeon, who is stressed by the restoration work execution, with precision and in a minimal delay considered safe to end the surgical procedure as a whole.

It is important to highlight that till now the elements of surgical steel tapes meet the main function they are designed to, which is to provide joining between separated parts of osseous structures after surgical procedures. Considering the negative aspect of ergonomics deficiency of applications, widely discussed on this application, it is conclusive that there is a maneuver margin to present efficient and constructive alternatives to such element in the field of surgical medicine.

Facing this challenge the applicant has idealized an evolutionary design of a metallic clamp that has resulted in a new product, wherein the constructive concept of steel tape elements receives a placement guide structure, which is based in the fixing to one edge of the main body of the tape. This guide structure presents in its free edge the assemblage of a needle, facilitating the access to the "sternum" bond region, for example, and making it easy the restoration procedure of this one.

### SUMMARY OF THE INVENTION

The present invention is a metallic clamp, also defined as metallic tape, of ordinary use during invasive surgical interventions, which receives a guide-wire element, more specifically a guide-wire element fixed to one edge of the metallic clamp, aiding in a decisive way the procedure of adequate placement close to osseous structure to be closed at the end of the surgical intervention.

The metallic clamp provides for ergonomics application, producing reliability gains for a surgical procedure as a whole. This invention brings in its core a technical and operational alternative to the use of steel tape elements known in the prior art.

This application provides a metallic clamp for surgical application comprising: (a) main body (1 A), including a first notch edge (1 B) and a second notch edge (1 C); (b) a metallic wire guide-structure (1 D) including a first and a second edge, the first edge attached to the second notch edge (1 C); and (c) a surgical needle (1 E) attached the second edge of the guide-structure (1 D).

This application also provides a method of using a metallic clamp for surgical application comprising the steps of: (i) introducing the surgical needle (1 E) in a first side of an osseous element to be restored, including at least a first side and a second opposite side; (ii) guiding the surgical needle (1 E) to the opposite side of the osseous element; (iii) introducing the surgical needle (1 E) in the opposite side of the osseous element to be treated; (iv) introducing the surgical needle (1 E), the metallic wire guide-structure (1 D) and the second notch edge (1 C) through the first notch edge (1 B); (v) jointing the notch edges (1 B) and (1 C) by means of applying a traction force to guide-structure (1 D); and (vi) cutting the surgical needle (1 E).

### BRIEF DESCRIPTION OF THE DRAWINGS

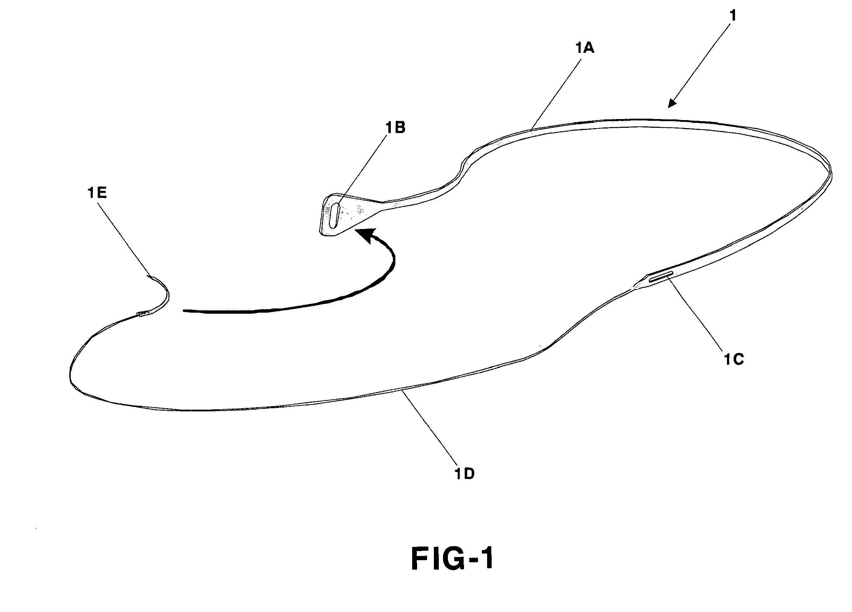

Figure 1 shows a perspective view of the metallic clamp immediately before applied around an osseous structure;

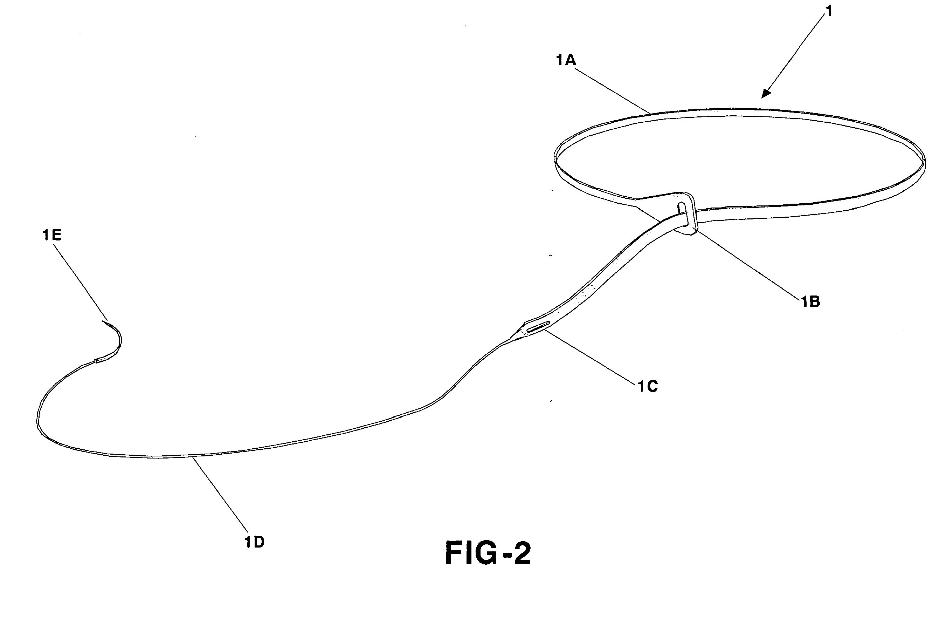

Figure 2 shows a perspective view immediately after fixed around an osseous structure.

A metallic clamp (1), comprising a main body (1A), provided with two edges to notch (1 B) and (1 C) respectively, providing a notch between both parts and a fit by pressing the main body (1 A) around the osseous structure during restoring procedure.

The clamp remains on the fixation of a guide-structure (1 D) to the notch edge (1 C) of the main body (1 A). A structure is formed by a metallic wire segment, preferably in surgical procedure steel, in order to assure asepsis during osseous structure restoring procedure and consequent finalization of surgical intervention.

A surgical needle of penetration (1E) is fixed on a free edge of the guide-structure (1 D) having two edges, acting as a facilitator agent of access to the "sternum" bone region, for example.

The operational procedure to the correct positioning of the metallic clamp (1), involved on the separated parts of the osseous element, is not complex, and the surgeon must solely introduce the needle (1 E) in one of the sides of an osseous element to be restored. The needle and the metallic wire will facilitate the duly guided passage to the opposite side of such osseous element, where the maneuver will be repeated. In sequence, the needle (1 E) is introduced through the notch edge (1 B), such operation involving the passage by this same notch edge of the full needle segment (1 E), including the notch edge passage (1 C).

The operator then proceeds with the pre-fit of the main body (1 A) to the osseous element through the traction of the guide-structure (1D) with handforce. The next step of the pre-fit of the main body (1 A) is the cut, with a pressure-cutter instrument, of the needle (1 E) letting the two notch edges (1 B) and (1 C) fitted and prepared to be conclusively joined, enabling the metallic clamp (1) to receive the final fit.

## Claims

1. A metallic clamp for surgical application comprising:
(a) a main body (1 A), including a first notch edge (1 B) and a second notch edge (1 C);
(b) a metallic wire guide-structure (1 D) including a first and a second edge, the first edge attached to the second notch edge (1 C), and
(c) a surgical needle (1 E) attached the second edge of the guide-structure (1 D).

2. A method of using a metallic clamp for surgical application according to Claim 1, comprising the steps of:
(i) introducing the surgical needle (1 E) in a first side of an osseous element to be restored, including at least a first side and a second opposite side;
(ii) guiding the surgical needle (1 E) to the opposite side of the osseous element;
(iii) introducing the surgical needle (1 E) in the opposite side of the osseous element to be treated;
(iv) introducing the surgical needle (1 E), the metallic wire guide-structure (1 D) and the second notch edge (1 C) through the first notch edge (1 B);
(v) jointing the notch edges (1 B) and (1 C) by means of applying a traction force to guide-structure (1 D); and
(vi) cutting the surgical needle (1 E).

3. The method according to Claim 2, wherein the step of cutting the surgical needle (1 E) is performed by a pressure-cutter instrument
